# EUROPÄISCHE PATENTSCHRIFT

(11) **EP 2 334 363 B1**
(45) Veröffentlichungstag und Bekanntmachung des Hinweises auf die Patenterteilung: **08.04.2015**
(21) Anmeldenummer: 09776112.6
(22) Anmeldetag: 26.08.2009
(51) Int. Cl.: A61M 5/315, A61M 5/31, A61M 25/01, A61M 25/06

(54) **VORRICHTUNG UND VERFAHREN ZUM ANLEGEN EINES ZUGANGS ZU EINEM HOHLORGAN**
DEVICE AND METHOD FOR CREATING AN ACCESS TO A HOLLOW ORGAN
DISPOSITIF ET PROCÉDÉ POUR L ÉTABLISSEMENT D'UN ACCÈS À UN ORGANE CREUX

(30) Priorität: 04.09.2008 DE 102008045692
(43) Veröffentlichungstag der Anmeldung: 22.06.2011
(73) Patentinhaber: Pobitschka, Walter, 61350 Bad Homburg (DE)
(72) Erfinder: Pobitschka, Walter, 61350 Bad Homburg (DE)
(74) Vertreter: Jendricke, Susann
(86) Internationale Anmeldenummer: PCT/DE2009/001190
(87) Internationale Veröffentlichungsnummer: WO 2010/031371

(56) Entgegenhaltungen:
- EP-A- 1 707 133
- WO-A-2008/008206
- DE-A1- 3 245 909
- DE-A1- 4 040 620
- DE-C1- 19 856 167
- US-A- 2 915 063
- US-A- 3 185 151
- US-A- 3 316 747
- US-A- 3 404 593
- US-A- 5 015 240

## Beschreibung

Die Erfindung betrifft eine Vorrichtung zum Anlegen eines Zugangs zu einem Hohlorgan mit einer Kanüle, mit einem Führungsdraht und mit einem Katheter.

Des weiteren betrifft die Erfindung ein Verfahren zum Anlegen eines Zugangs zu einem Hohlorgan, wobei mittels einer Kanüle einer Spritze der Hohlkörper punktiert wird, wobei die Kanüle und die Spritze nach der Punktierung getrennt werden, wobei ein Führungsdraht durch die Kanüle in den Hohlkörper eingebracht wird, wobei ein Katheter entlang des Führungsdrahtes in das Gefäß eingebracht wird und wobei der Führungsdraht nach Katheterlegung entfernt wird.

Schließlich wird ein spezielles Werkzeug beschrieben, das die Erfindung nicht darstellt und die gemäß der Vorrichtung verwendete Spritze vom Katheter abtrennt.

Katheter sind Röhrchen oder Schläuche verschiedener Durchmesser aus Kunststoff, Latex, Silikon, Metall oder Glas, mit denen Hohlorgane wie Gefäße oder Harnblase usw. sondiert, entleert, gefüllt oder gespült werden können. Die vorliegende Erfindung richtet sich besonders auf das Anlegen eines Dauerzugangs in ein Blutgefäß, schließt aber sämtliche Bereiche der Katheterisierung mit ein.

Ein Dauerzugang wird für die Gabe von Dauerinfusionen und wiederholte Injektionen in das venöse System eines Organismus benötigt. Wenn in der Praxis ein Dauerzugang gelegt wird, wird zunächst mittels der Kanüle einer mit Kochsalzlösung halbgefüllten Spritze das Gefäß punktiert, danach werden die Kanüle und die Spritze voneinander getrennt. Durch die Kanüle wird ein Führungsdraht in das Gefäß bis zur vorgegebenen Position des Katheterauslasses vorgeschoben, entlang dessen ein Katheter in das Gefäß eingebracht wird. Vor der Katheterlegung wird die Kanüle ausgefädelt und es kann danach auch eine Gefäßeingangserweiterung stattfinden, indem ein Dilatator entlang des Führungsdrahtes bis in den Eingangsbereich des Gefäßes geschoben wird und diesen erweitert. Nach der Erweiterung wird der Dilatator entfernt und es erfolgt die Einführung des Katheters in das Gefäß. Nach der Katheterlegung wird der Führungsdraht entfernt. Der Führungsdraht wird die gesamte Zeit über in der Hand gehalten - zunächst vor dem Katheter, danach hinter dem Auslass des Katheters.

Die einzelnen Einrichtungen - Spritze, Führungsdraht, Kanüle, Dilatator, Katheter - werden in der Regel in einem Behandlungszimmer in unsteriler Atmosphäre gehandhabt. Es besteht Infektionsgefahr. Gerade im Behandlungszimmern ist die Konzentration an Bakterien und Viren dort ein und ausgehender Patienten groß, was gerade für geschwächte Organismen zur Gefahr werden kann.

Ausgehend von dem aus der Praxis bekannten Stand der Technik liegt der Erfindung die Aufgabe zugrunde, eine Vorrichtung und ein Verfahren zum Anlegen eines Zugangs zu einem Hohlorgan anzugeben, wobei das Kontaminationsrisiko vermindert wird. Das spezielle Werkzeug, das die Erfindung nicht darstellt, dient zur Entfernung der Spritze vom Katheter, nachdem der Zugang gelegt ist.

Die voranstehende Aufgabe wird im Hinblick auf die Vorrichtung durch die Merkmale des Patentanspruches 1 gelöst. Danach ist eine Vorrichtung der in Rede stehenden Art derart ausgestaltet, dass die Kanüle Bestandteil einer Spritze mit einer Durchtrittsöffnung für den Führungsdraht und den Katheder ist und dass die Spritze, der Katheter und der Führungsdraht innerhalb eines geschlossenen Systems in Form zumindest einer Umhüllung angeordnet sind und zwar derart, dass die Vorrichtung von aussen betätigbar ist, und dass die Umhüllung durch die Kanüle perforierbar ist.

Danach ist das spezielle Werkzeug das die Erfindung nicht darstellt zur Abtrennung der Spritze der erfindungsgemäßen Vorrichtung nach dem Anlegen des Zugangs zum Hohlorgan vom Katheter ausgestattet mit zwei über ein Scharnier verbundenen Backen zum Umgreifen der Spritze und mit Zerkleinerungseinrichtungen an der Innenseite der Backen, wobei die Backen Bestandteil einer Zange sind und über je einen Zangenhebel aufeinander zu oder voneinander weg bewegbar sind.

Danach ist ein Verfahren, das die Erfindung nicht darstellt der in Rede stehenden Art derart ausgestaltet, dass der Führungsdraht durch eine Durchtrittsöffnung der Spritze zur Kanüle gelangt, dass die Spritze, der Katheter und der Führungsdraht innerhalb eines geschlossenen Systems, insbesondere innerhalb zumindest einer Umhüllung, enthalten sind, wobei deren Betätigung von außen erfolgt und dass die Kanüle vor der Punktierung des Hohlorgans die Umhüllung perforiert.

Ausgehend von dem aus der Praxis bekannten Stand der Technik ist erkannt worden, dass das Anlegen eines Zugangs zu einem Hohlkörper ernstzunehmende Kontaminationsrisiken birgt. Erfindungsgemäß ist erkannt worden, dass die Kontaminationsgefahr verringert werden kann, wenn die erforderlichen Einzelteile innerhalb eines geschlossenen Systems angeordnet sind. Das geschlossene System wird durch mindestens eine Umhüllung ausgebildet, die allerdings mittels der Kanüle perforierbar ist. Durch die Abschirmung der Einzeleinrichtungen gegen die Umwelt, wird eine Kontamination mit Erregern aus der Umwelt ausgeschlossen solange das System geschlossen ist. Erst beim Einstich in den Organismus wird das System geöffnet, wobei die Umhüllung von der Kanüle kurzerhand durchstochen wird. Die Zeit, innerhalb der die Kanüle mit der Umgebungsluft in Berührung kommt, ist somit sehr gering. Es ist offensichtlich, dass in erfindungsgemäßer Weise das Kontaminationsrisiko verringert wird. Bezüglich des speziellen Werkzeuges als Ergänzung zur Vorrichtung ist hervorzuheben, dass die dortige Spritze nach der Katheterisierung keine Funktion mehr hat und als Ballast entfernt werden kann.

Damit der Zuführdraht und der Katheter an die gewünschte Position im Hohlkörper bzw. in einem Blutgefäß gelangen kann, könnte sich die Durchtrittsöffnung durch den Kolben der Spritze erstrecken und mit der kanülenseitigen Durchtrittsöffnung des Tubus der Spritze korrespondieren.

Da die Vorrichtung über mehrere Bestandteile verfügt, könnten diese in separaten Umhüllungen angeordnet sein, die wegen des Transfers von Vorrichtungsteilen zum Einsatzort untereinander in Verbindung gebracht werden können. Insbesondere sind die Umhüllung der Spritze, die Umhüllung des Zuführbereiches für den Führungsdraht und den Katheter zu nennen, die voneinander getrennt und miteinander verbunden werden können. Im Hinblick auf die Öffnungsstelle der Kanülenumhüllung ist die Verschließbarkeit notwendig, damit die Einzelteile der Vorrichtung nicht durch die eintretende Außenluft kontaminiert werden. Eine separate Umhüllung könnte auch dort vorgesehen sein, wo die Spritze geöffnet oder verschlossen wird, insbesondere für die Halteeinrichtung der Kanüle und für die Verschlusseinrichtung am Ende des Spritzenkolbens. Durch die separate Umhüllung kann die Halteeinrichtung gedreht werden, ohne dass dies einen Einfluss auf die Umhüllung der Spritze oder die Kanülenumhüllung hat. Bei einem Ausführungsbeispiel, wobei in einer Umhüllung sowohl die Spritze als auch die Halteeinrichtung enthalten ist, könnte es zur unnötigen Beanspruchung des für die Spritze vorgesehenen Bereiches der Umhüllung kommen. Zudem ist man mit einer separaten Umhüllung für die Halteeinrichtung freier in der Gestaltung der Spritzenumhüllung, die am Tubusteil sehr eng anliegen soll.

Die Umhüllungen könnten aus einem weichen und reißfesten Kunststoff bestehen, der transparent oder zumindest semitransparent ist und eine Handhabung der Vorrichtungsteile von außen ermöglicht. Im Perforierungsbereich der Kanüle sollte die Umhüllung aus einem leicht durchdringbaren Material, wie bspw. Latex, bestehen.

Außerdem könnte an der Spritze ein Sterilpflaster vorgesehen sein, welches zur Abdeckung der Einstichstelle dient. Mit dem Sterilpflaster wird weiter unterstützt, die Einstichstelle minimalstem Kontakt mit der Atmosphäre auszusetzen. Das Sterilpflaster kann direkt nach dem Einstich aufgebracht werden oder auch nachdem der Katheter gelegt wurde.

Bevor die erfindungsgemäße Vorrichtung zum Einsatz kommt, könnten die Kanüle und die dortige Umhüllung zusätzlich innerhalb einer entfernbaren Schutzhülse angeordnet sein. Auf diese Weise wird das empfindliche vordere Ende der leicht perforierbaren Umhüllung vor mechanischer Einwirkung und Auflösung des geschlossenen System geschützt.

Die Umhüllung der Kanüle könnte im Bereich der Kanüle als Faltenbalg ausgebildet sein, die nach der Perforation zurückgeschoben werden kann, so dass die Kanüle freiliegt und die übliche Handhabung erfolgen kann.

Zur Aufweitung des Gefäßeinganges könnte die Spritze einen Dilatator umfassen. Dieser könnte in zweckmäßiger Weise lösbar mit dem Tubus der Spritze verbunden sein. Bspw. könnte am Tubus ein Ansatz mit einem Innengewinde vorgesehen sein in das der Dilatator über sein Außengewinde eingeschraubt werden kann. Die Aufweitung des Hohlkörpereingangs ist erforderlich, damit der Katheter eingeführt werden kann. Die Aufweitung kann durch Gleiten des Dilatators über die Kanüle - wenn diese sich noch im punktierten Hohlkörper befindet - erfolgen oder aber auch nach Entsorgung der Kanüle durch Gleiten entlang des Führungsdrahtes.

Des weiteren könnte am Tubus der Spritze eine Halteeinrichtung für die Kanüle vorgesehen sein, die über die Haltefunktion hinaus auch eine Verschluss- und Öffnungsfunktion wahrnimmt. Diese könnte auf verschiedene lichte Weiten eingestellt werden, zum einen, um zunächst die Kanüle entsprechend ihrem Querschnitt dicht und fest zu halten, zum anderen, um die Durchtrittsöffnung für die hindurchzuführenden Vorrichtungsteile, wie Zuführdraht und Katheter, auszubilden. Auch die Halteeinrichtung könnte eine separate Umhüllung aufweisen. An diese Umhüllung könnte sich die Umhüllung der Spritze anschließen. Im Hinblick auf die Bewegung des Kolbens im Tubus könnte auch dessen Umhüllung zumindest teilweise als Faltenbalg ausgebildet sein.

Am Ende des Kolbens der Spritze könnte eine Verschlusseinrichtung vorgesehen sein, die die Durchtrittsöffnung freigibt oder verschließt und die vorzugsweise auf verschieden Querschnitte des Führungsdrahtes und des Katheters einstellbar ist. Konstruktiv könnte dies durch eine dicht verschließbare Blende realisiert werden, die beim Auf- und Zudrehen entsprechend der Drehstellung den Ein- bzw. Auslass der Verschlusseinrichtung mehr oder weniger freigibt. Es versteht sich, dass die gewünschte Blendenstellung arretierbar ist. Alternativ zur Integration der Verschlusseinrichtung in die Spritzenumhüllung könnte wie bei der Halteeinrichtung eine separate Ummantelung auch bezüglich der Verschlusseinrichtung vorgesehen sein, um den Verdrehvorgang beim Öffnen oder Verschließen des Verschlusses mittels Rändelring abzukoppeln.

Auch für den an die Spritze angrenzenden Teil der Vorrichtung, der den Führungsdraht und den Katheter enthält, könnte eine Umhüllung vorgesehen sein. Da es hier besonders in der Handhabungszone beim Einfädeln des Führungsdrahtes in die Durchtrittsöffnung der Spritze, auf eine bequeme Handhabung ankommt, könnte die Umhüllung insgesamt als Faltenbalg ausgeführt sein.

Überall dort, wo die Umhüllungen geschlossen bleiben, nämlich im Bereich des Spritzenkolbens und im Bereich der Zuführung von Führungsdraht und Katheter örtlich vor der Spritze, können Volumenänderungen des enthaltenen Mediums auftreten. Die Geschlossenheit des Systems, abgesehen von der einzigen Perforationsstelle, kann gewahrt werden, wenn die Druckdifferenzen ausschließlich innerhalb des Systems ausgeglichen werden Hierzu könnte eine Druckausgleicheinrichtung vorgesehen sein, die mit mindestens einem System steril strömungsverbunden ist. Mit anderen Worten: Verdrängte Luft wird in vorteilhafter Weise nicht in die Umgebungsluft entlassen, sondern in einer Druckausgleicheinrichtung aufgefangen und steht dort zur Verfügung, damit kein Vakuum bei anderen Vorgängen entsteht. Die erfindungsgemäße Vorrichtung kann direkt vor Ort am Patienten angewendet werden und als Einmalartikel ausgebildet sein. Standardisierte industriell gefertigte Einmalartikel gemäß der Erfindung bereichern das Arbeiten unter Bedingungen einer stark verminderten Kontamination enorm.

Damit auch die Zuführung des Führungsdrahtes und des Katheters innerhalb des geschlossenen Systems stattfindet, könnten diese innerhalb eines Vorratsbehältnisses enthalten sein, der sich an die Umhüllung für den Führungsdraht und den Katheter anschließt. Dieses Vorratsbehältnis ist vorzugsweise steril angekoppelt, und gewährleistet die Verbindung zur Umhüllung, in der das Handling der Vorrichtungsteile erfolgt. Wenn der Rückbau der Vorrichtung einsetzt, kann das Vorratsbehältnis seine Funktion ändern und zum Entsorgungsbehältnis werden. Von besonderem Vorteil für eine bequeme Handhabung ist es, wenn der Katheter innerhalb des Vorratsbehältnisses mit dem Draht vorkonfektioniert und auf diesen aufgezogen ist.

Zwischen der Umhüllung für den Führungsdraht und den Katheter und dem Vorrats- und Entsorgungsbehältnis könnte ein Verteiler für diverse Anschlüsse vorgesehen sein. Diese Anschlüsse könnten der Zufuhr von Medikamenten dienen oder auch der Zufuhr von physiologischer Kochsalzlösung zur Aufrechterhaltung einer Flüssigkeitssäule im Katheter. Wesentlich ist, dass die Anschlüsse abgedichtet am Verteiler angeordnet sind, so dass die Geschlossenheit des Systems nicht unterbrochen wird. Die Anschlüsse könnten bspw. angeschweißt sein.

Von besonderem Vorteil ist die Zuordnung steril ummantelter Ventile zu den einzelnen Anschlüssen. Diese verhindern den Rückfluss von aus dem Hohlorgan möglicherweise austretender Flüssigkeiten, die einen Nährboden für Mikroorganismen ausbilden könnten. Nur bis zum Ventil bzw. Absperrhahn kann ein Rückfluss stattfinden, der zu pflegende Abschnitt des Katheters wird reduziert und vereinfacht. Der Vorteil der ummantelten Ventile ergibt sich auch in umgekehrter Richtung, bspw. dann, wenn der Zugang mit Alkohol gereinigt wird. Dann kann das Reinigungsmittel oder auch kontaminierte Luft keinesfalls in den Katheter gelangen. Genauso ist dadurch auch eine dosierte Abgabe eines zugeführten Medikamentes möglich.

Die Umhüllung könnte einen steril konnektierbaren Anschluss für ein Pumpsystem aufweisen. Dies ist vorteilhaft, um während der Manipulation im Inneren der Umhüllung einen leichten Überdruck durch sterile Luft zu erzeugen, der in einer gerichteten Luftströmung resultiert. Der Einsatz des Pumpsystems kann besonders dann zum Einsatz kommen, wenn die Kanüle entfernt wird und in Richtung Vorrats- und Entsorgungsbehältnis bewegt wird. Dann ist die Halteeinrichtung geöffnet und kontaminierte Luft könnte unerwünscht in die Vorrichtung, insbesondere in den Katheter gelangen. Mittels der eingepumpten sterilen Luft wird quasi eine Druckwand aufgebaut, die das Einströmen von kontaminierter Luft verhindert. Dieser Vorgang dauert nur kurz an. Ein Druckabbau kann über die Druckausgleicheinrichtung erfolgen. Dieser zusätzliche Aufwand ist für die Anwendung bei besonders kontaminationsgefährdeten Patienten angebracht.

Nachdem der Führungsdraht in den Hohlkörper eingebracht ist, geht es darum, die Kanüle aus dem Organismus zu entfernen. Der Kolben der Spritze befindet sich in eingefahrener Position bis zum Auslass des Tubus, da er bei der Punktierung des Hohlorgans, insbesondere des Gefäßes bereits von außen über die Umhüllung betätigt wurde. Der Katheter wird in den Tubus bis zu deren Auslass im Bereich der Halteeinrichtung vorgeschoben. Bei mehrlumigen Kathetern muss ein zentrales Lumen unmittelbar im Zentrum der Durchtrittsöffnung des Kolbens positioniert sein. Die Halteeinrichtung für die Kanüle wird geöffnet, so dass die Kanüle durch den Katheter zum inzwischen entleerten Vorrats- und Entsorgungsbehältnis verbracht werden kann.

Nach einer besonders bevorzugten Ausgestaltung der Erfindung könnte die Kanüle magnetisiert sein und mit einer Magneteinrichtung zusammenwirken. Auf diese Weise gelingt ein berührungsfreier Transport der Kanüle nach dem Öffnen der Halteeinrichtung durch den Katheter hindurch zum Vorrats- und Entsorgungsbehältnis, welches dann nur noch Entsorgungsfunktion hat. Die Magneteinrichtung könnte als Ringmagnet oder in Form eines aufklappbaren Magneten ausgeführt sein. Die Stärke des Magnetfeldes könnte durch einen Dauermagneten erzielt werden. Auch eine Magneteinrichtung unter Ausnutzung elektrischer Magnetfelder ist möglich.

Wenn die Kanüle entsorgt ist, erfolgt die Positionierung des Katheteres durch die geöffnete Halteeinrichtung hindurch, entlang des Führungsdrahtes im Hohlorgan an der gewünschten Stelle. Danach wird der Führungsdraht über den Faltenbalg der Umhüllung für Zuführdraht und Katheter hinausgezogen und im Vorrats- und Entsorgungsbehältnis platziert, welches dann nur noch Entsorgungsfunktion hat und nun steril abgetrennt werden kann.

Danach wird die Eintrittstelle des Katheters in den Organismus steril versorgt. Hierzu ist zunächst das Sterilpflaster zu lösen, um es danach - nach entsprechender Desinfektion - erneut wieder zur Abdeckung zu verwenden. Alternativ kann das Sterilpflaster auch erstmals spätestens jetzt zum Einsatz kommen und die vorübergehende Fixierung übernehmen, bis die endgültige Fixierung nach herkömmlicher Weise erfolgt. Nach einer weiteren Alternative könnten auch mindestens zwei Sterilpflaster vorgesehen sein, so dass die erste Garnitur direkt nach Punktierung bspw. eines Blutgefäßes auf die Haut aufgebracht wird, zur Katheterisierung entfernt wird und dann nach Katheterisierung und nach der Entfernung des Führungsdrahtes die zweite Garnitur Sterilpflaster über die Eintrittsstelle gelegt wird.

Nachdem die Kathetereintrittstelle steril versorgt ist, befindet sich die Spritze mit Tubus, Kolben und Dilatator noch a Katheter. Dies ist für den Patienten unbequem. Als Beispiel, das die Erfindung nicht darstellt, wird ein Werkzeug beschrieben, mit dem die auf dem Katheter sitzende und nunmehr, nachdem der Zugang gelegt wurde, nutzlose Spritze abgetrennt werden kann. Das spezielle Werkzeug könnte wie eine Zange gehandhabt werden und die Brechbacken könnten vorzugsweise in Form von Formteilen vorliegen, die auf den Tubus und den Kolben der Spritze sowie auf die Halteeinrichtung für die Kanüle und die Verschlusseinrichtung am Kolbenende genau abgestimmt sind. Die Spritze könnte aus einem leicht zerstörbaren Material, insbesondere aus Kunststoff, bestehen, das bspw. durch Metallzähne leicht zerkleinert werden kann. Das Werkzeug könnte des weiteren eine Auffangeinrichtung für die Bruchstücke der zerstörten Spritze aufweisen. In den Backen könnten Öffnungen vorgesehen sein, durch die die Bruchstücke der zerstörten Spritze in die Auffangeinrichtung gelangen.

Bezüglich des Verfahrens, das die Erfindung nicht darstellt, wird ausgeführt, dass nach der Punktierung des Hohlorgans, insbesondere eines Blutgefäßes, wie üblich der Führungsdraht im Blutgefäß positioniert wird. Neu ist hierbei, dass der Führungsdraht aus einem Vorrats- und Entsorgungsbehältnis durch die Durchtrittsöffnung des während der Punktierung bis zum Tubusauslass eingefahrenen Spritzenkolbens und die Kanüle hindurchgeführt wird, wobei die Handhabung von außen geschieht, da sich alles im Inneren eines Systems abspielt. Nach der Positionierung des Führungsdrahtes wird der Katheter durch die Durchtrittsöffnung der Spritze zunächst bis zum Tubus vorgeschoben. Danach wird die magnetisierte Kanüle aus einer Halteeinrichtung entfernt und mittels Magneteinrichtung durch den Katheter hindurch zu einem Vorrats- und Entsorgungsbehältnis, dessen Funktion sich zu diesem Zeitpunkt auf die Entsorgung beschränkt. Wie bereits im Zusammenhang mit der erfindungsgemäßen Vorrichtung ausgeführt, könnte über ein an die Umhüllung angeschlossenes Pumpsystem sterile Luft oder ein Inertgas in die Umhüllung eingebracht werden, insbesondere dann, wenn die Kanüle aus der Halteeinrichtung entfernt wird und kontaminierte Luft aus dem Bereich zwischen Tubus und Hohlorgan in die Umhüllung der Spritze bzw. in den Katheter eindringen könnte. Ein Druckausgleich findet generell innerhalb des Systems dort statt, wo sich das Volumen innerhalb der Umhüllung ändert.

Nach der Entsorgung der Kanüle im Vorrats- und Entsorgungsbehältnis wird der Katheter durch die geöffnete Halteeinrichtung am Tubus hindurch entlang des Führungsdrahtes in das Hohlorgan vorgeschoben. Nach der Katheterisierung wird der Führungsdraht aus dem Hohlorgan herausgezogen und in das Vorrats- und Entsorgungsbehältnis verbracht. Auch dabei kann sterile Luft eingepumpt werden, obgleich die auf den Querschnitt einstellbare Durchtrittsöffnung der Halteeinrichtung aufgrund des sehr kleinen Drahtquerschnitts nur sehr geringe Kontaminationsgefahr birgt.

Das Vorrats- und Entsorgungsbehältnis wird nach der Aufnahme der verbrauchten Kanüle und des verbrauchten Führungsdrahtes vom Anschluss steril abgeschweißt. Wie im Zusammenhang mit dem erfindungsgemäßen Werkzeug beschrieben, wird die Spritze mittels speziellem Werkzeug entfernt, um dem Patienten Bequemlichkeit zu ermöglichen. Aus dem gleichen Grund werden auch die Reste der Umhüllung zwischen der Spritze und dem Anschluss für das Vorrats- und Entsorgungsbehältnis entfernt, was mittels Schere erfolgen kann. Alternativ könnten auch Abreißeinrichtungen vorgesehen sein, die es gestatten, das Material der Umhüllung entlang vorgegebener Soll-Reißstellen zu entfernen. Zum Schluss bleibt nur noch der Katheter übrig, ggf. mit Verteiler und mehreren Anschlüssen.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung eines Ausführungsbeispiels der Erfindung anhand der Zeichnung zu verweisen. In Verbindung mit der Erläuterung des angeführten Ausführungsbeispiels der Erfindung werden auch im allgemeinen bevorzugte Ausgestaltungen und Weiterbildungen der Lehre erläutert. In der Zeichnung zeigen
- Fig. 1: in schematischer Darstellung eine Seitenansicht der erfindungsgemäßen Vorrichtung in Ruhestellung,
- Fig. 2: in schematischer Darstellung, vergrößert, ein Detail aus Fig. 1, betreffend die Schutzhülse,
- Fig. 3: in schematischer Darstellung, vergrößert, teilweise den Gegenstand aus Fig. 1, nach Entfernung der Schutzhülse,
- Fig. 4: in schematischer Darstellung, vergrößert, teilweise den Gegenstand aus Fig. 1, nach Durchstechen der Umhüllung im Perforationsbereich und Punktierung des Blutgefäßes,
- Fig. 5: in schematischer Darstellung, vergrößert, teilweise den Gegenstand aus Fig. 1, während des Einbringens des Führungsdrahtes durch die Spritze und die Kanüle hindurch in das Blutgefäß,
- Fig. 6: in schematischer Darstellung, vergrößert, teilweise den Gegenstand aus Fig. 1, während des Einsatzes des Dilatators,
- Fig. 7: eine Prinzipskizze zur Handhabung des Dilatators,
- Fig. 8: in schematischer Darstellung, vergrößert, teilweise den Gegenstand aus Fig. 1, nach dem Einsatz des Dilatators
- Fig. 9: in schematischer Darstellung, vergrößert, teilweise den Gegenstand aus Fig. 1, mit dem bis zur geschlossenen Halteeinrichtung am Tubus, durch die Spritze hindurch herangeführten Katheter,
- Fig. 10: in schematischer Darstellung, vergrößert, teilweise den Gegenstand aus Fig. 1, während des Entfernens der Kanüle durch den Katheter hindurch mittels Magneteinrichtung,
- Fig. 11: in schematischer Darstellung, vergrößert, teilweise den Gegenstand aus den Fig. 1 und 11, betreffend den Weg der Kanüle zum Vorrats- - und Entsorgungsbehältnis,
- Fig. 12: in schematischer Darstellung, vergrößert, teilweise den Gegenstand aus den Fig. 1, während des Einbringens des Katheters durch die Spritze hindurch in das vorbereitete Blutgefäß,
- Fig. 13: in schematischer Darstellung, vergrößert, teilweise den Gegenstand aus den Fig. 1, während des Herausziehens des Zuführdrahtes aus dem Blutgefäß nach abgeschlossenem Einbringens des Katheters,
- Fig. 14: in schematischer Darstellung, vergrößert, teilweise den Gegenstand aus Fig. 1, nach abgeschlossenem Anlegen des Zugangs
- Fig. 15: in schematischer Darstellung, eine Skizze eines Spezialwerkzeuges zur Trennung von Spritze und Katheter, das die Erfindung nicht darstellt und
- Fig. 16: in schematischer perspektivischer Darstellung, teilweise den Gegenstand aus Fig. 15.

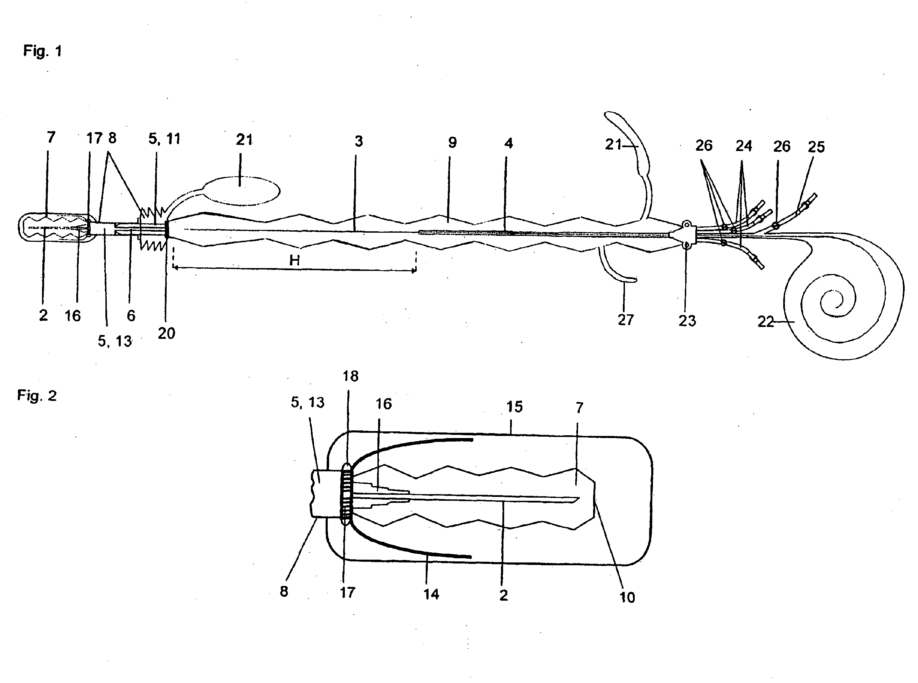

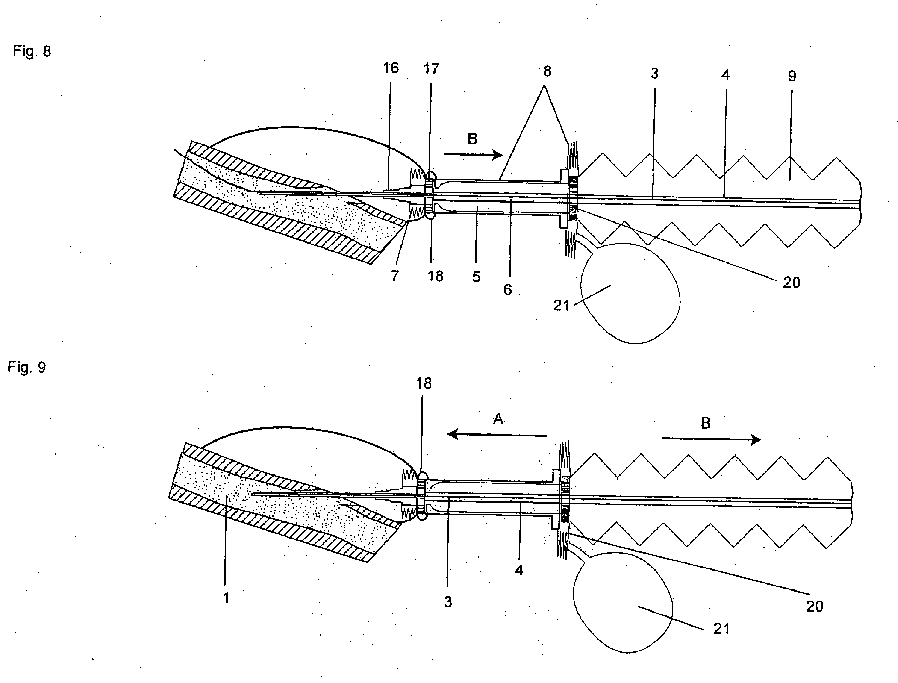

Aus den Fig. 1 bis 14 ergibt sich eine Vorrichtung zum Anlegen eines Zugangs zu einem Hohlorgan in Form eines Blutgefäßes 1 mit einer Kanüle 2, mit einem Führungsdraht 3 und mit einem Katheter 4. Die Kanüle 2 ist Bestandteil einer Spritze 5.

Erfindungsgemäß ist die Spritze 5 mit einer Durchtrittsöffnung 6 für den Führungsdraht 3 und den Katheter 4 ausgestattet. Die Spritze 5, der Katheter 4 und der Führungsdraht 3 sind gemäß Fig. 1 innerhalb eines geschlossenen Systems angeordnet. Das geschlossene System umfasst mehrere luftdichte, transparente und reißfeste Umhüllungen 7, 8, 9 aus Kunststoff, über die die Spritze 5, der Führungsdraht 3, die Kanüle 2 und der Katheter 4 von außen betätigt werden können. Im vorliegenden Ausführungsbeispiel ist die Umhüllung 7 im Perforierungsbereich 10 durch die Kanüle 2 perforierbar. Im Perforierungsbereich 10 besteht die Umhüllung 7 aus Latex.

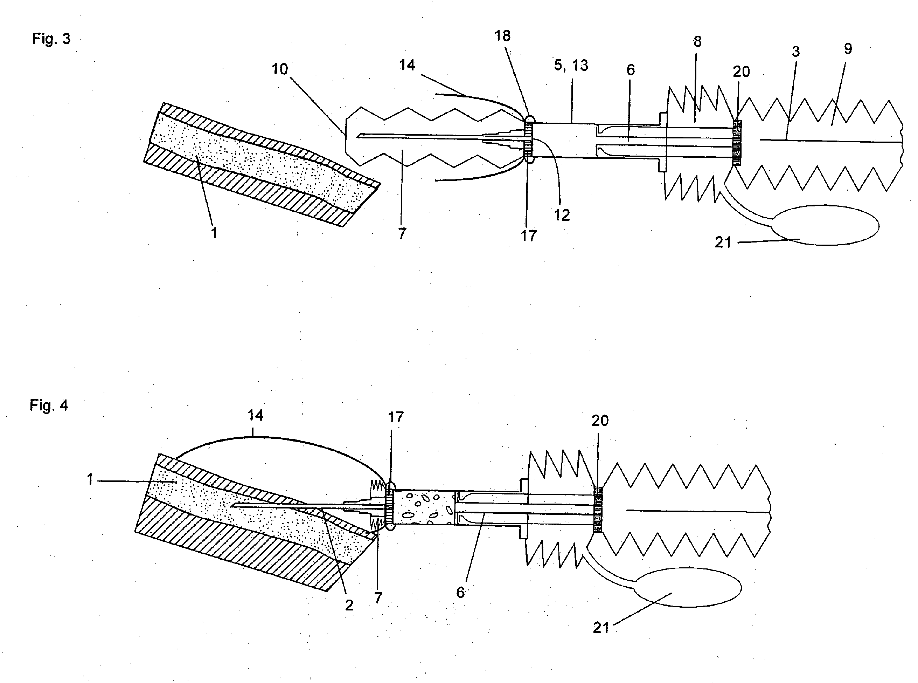

Die Durchtrittsöffnung 6 erstreckt sich durch den Kolben 11 der Spritze 5 und korrespondiert in eingefahrener Stellung - vgl. Fig. 4 bis 6, 8 bis 10 mit der kanülenseitigen Durchtrittsöffnung 12 des Tubus 13 der Spritze 5.

An der Spritze 5 ist ein Sterilpflaster 14 vorgesehen ist, welches zur Abdeckung der Einstichstelle dient. In Fig. 2 ist gezeigt, dass die Kanüle 2 und die dortige Umhüllung 7 während des Ruhezustandes innerhalb einer Schutzhülse 15 angeordnet sind. Die Umhüllung 7 im Bereich der Kanüle 2 liegt in Form eines Faltenbalgs vor, der nach der Perforation in Richtung des Tubus 13 zurückschiebbar ist.

Die Spritze 5 umfasst des weiteren einen Dilatator 16, der an der Halteeinrichtung 17 für die Kanüle 2 angeordnet ist. Die Halteeinrichtung 17 ist dem Tubus 13 der Spritze 5 zugeordnet und von einer eigenen Umhüllung 18 umgeben. Die Halteeinrichtung 17 verfügt über einen Rändelring, der von außen über die Umhüllung 18 betätigt wird und wobei eine hier nicht gezeigte Blende auf verschiedene Querschnitte, nämlich auf die Querschnitte der Kanüle 2, des Führungsdrahtes 3 und des Katheters 4 einstellbar ist. Die separate Umhüllung 18 wurde hier gewählt, um Verdrehungen mit dem Rändelring der Halteeinrichtung 17 gegenüber der Umhüllung 8 der Spritze 5 wirkungslos zu gestalten.

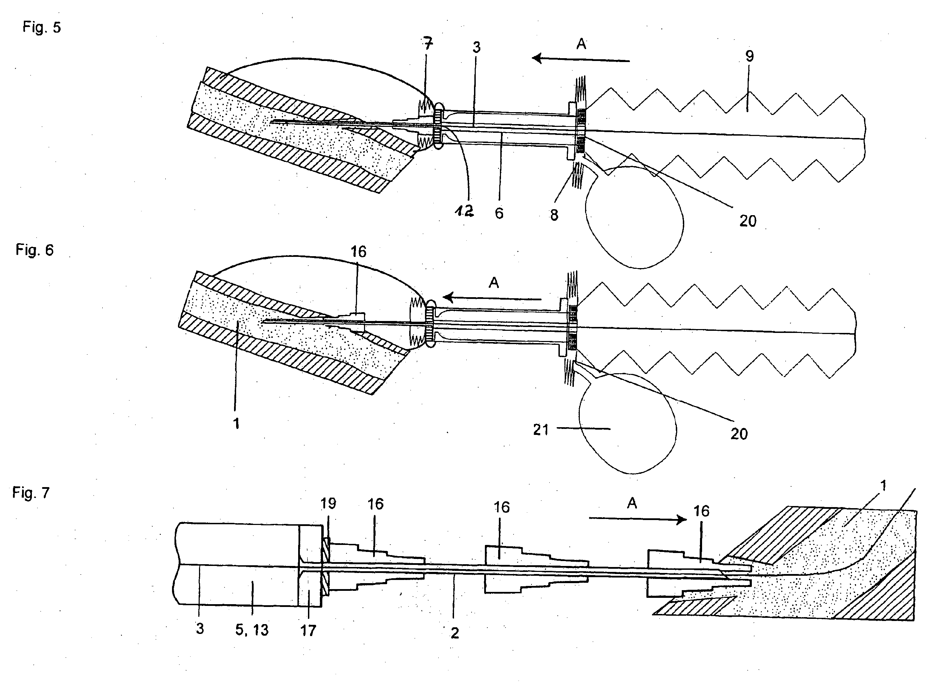

In den Fig. 6 bis 8 ist gezeigt, dass der Dilatator 16 an der Halteeinrichtung 17 lösbar befestigt ist. Mit 19 ist in Fig. 7 ein Gewindebauteil bezeichnet, in das der Dilatator 16 eingeschraubt ist. Zum Einsatz an der Einstichstelle wird der Dilatator 16 vom Gewindebauteil 19 gelöst. Nach der Erweiterung des Eingangs wird der Dilatator 16 wieder am Gewindebauteil 19 befestigt.

Die Umhüllung 8 der Spritze 5 ist im Bereich ihres Kolbens 11 als Faltenbalg ausgebildet, während sie sich im Bereich des Tubus 13 glatt an die Tubuswandung anlegt und eine möglichst normale Betätigung der Spritze 5 ermöglicht.

Die Umhüllungen 8 und 9 können untereinander in Verbindung gebracht werden, was zur Durchführung des Verfahrens auch unbedingt erforderlich ist. Dazu ist am Ende des Kolbens 11 der Spritze 5 eine Verschlusseinrichtung 20 vorgesehen, die die Durchtrittsöffnung 6 freigibt oder verschließt und dabei auf verschieden Querschnitte des Führungsdrahtes 3 und des Katheters 4 einstellbar ist und somit auch eine Zentrierungsfunktion erfüllt.

Die Umhüllung 9 für die Zuführung von Führungsdraht 3 und Katheter 4 sowie für die spätere Abführung des Führungsdrahtes 3 ist ebenfalls als Faltenbalg ausgeführt und schließt sich an die Spritze 5 an. Mit H ist die Handhabungszone der Umhüllung 9 bezeichnet. Dort erfolgt das Einfädeln des Führungsdrahtes und des Katheters 4 durch die Verschlusseinrichtung 20 in die Durchtrittsöffnung 6 und von dort durch die Halteeinrichtung 17 weiter zum Blutgefäß 1. Die Umhüllungen 8 und 9 umfassen jeweils eine Druckausgleicheinrichtung 21.

An die Umhüllung 9 schließt sich ein Vorrats- und Entsorgungsbehältnis 22 an, aus dem Führungsdraht 3 und Katheter 4 zur Spritze 5 und schließlich zum Blutgefäß 1 führbar sind. Im vorliegenden Ausführungsbeispiel sind der Katheter 4 und der Führungsdraht 3 vorkonfektioniert, d. h. der Katheter 4 innerhalb des Vorrats- und Entsorgungsbehältnisses 22 ist bereits auf den Führungsdraht 3 aufgezogen.

Zwischen der Umhüllung 9 und dem Vorrats- und Entsorgungsbehältnis 22 ist ein Verteiler 23 für diverse Anschlüsse 24, 25 für den Katheter 4 vorgesehen. Die Anschlüsse 24, 25 sind bspw. mit Infusionsbestecken oder anderen Kopplungseinheiten konnektierbar und umfassen jeweils ein steril ummanteltes Ventil 26. Der Anschluss 25 wird erst aktiviert, wenn das Vorrats- und Entsorgungsbehältnis 22 vom System abgekoppelt ist. Der Anschluss 25 dient zur Beschickung des zentralen Lumens des hier vierlumigen Katheters 4. Die Anschlüsse 24 beliefern die drei weiteren Lumen des Katheters 4. Das Vorrats- und Entsorgungsbehältnis 22 stellt quasi eine Erweiterung des zentralen Lumens des Katheters 4 dar.

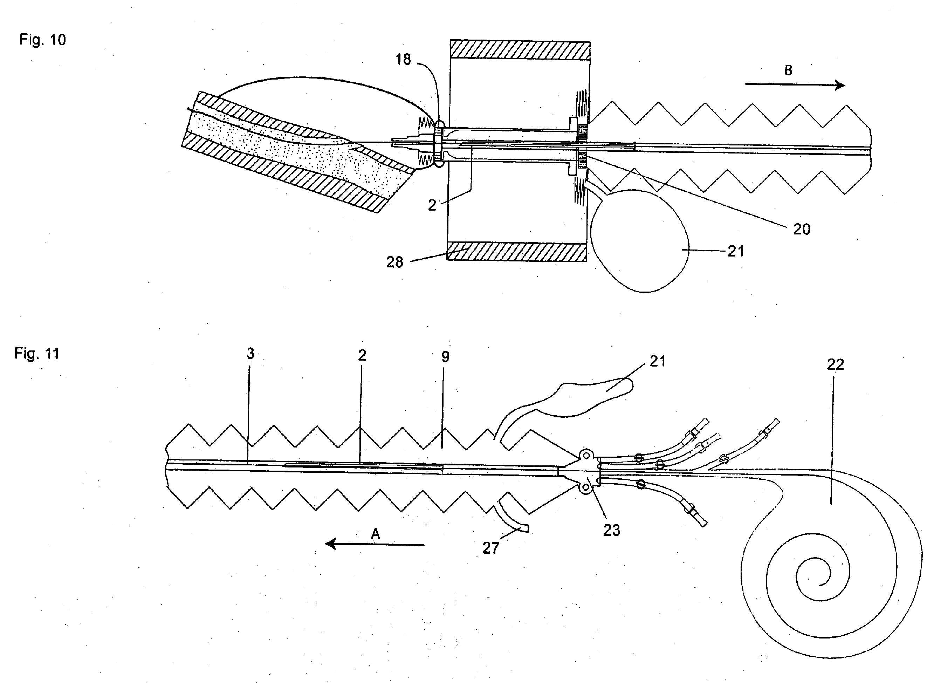

Die Umhüllung 9 weist außerdem einen steril konnektierbaren Anschluss 27 für ein hier nicht dargestelltes Pumpsystem auf, mit dem sterile Luft in die Umhüllung 9, ggf. auch in die Umhüllung 8 eingeführt wird, um eine Sterilfront gegen kontaminierte Luft von außen aufzubauen, die eindringen könnte, wenn die Halteeinrichtung 17 geöffnet wird, insbesondere dann, wenn die Kanüle 2 in Richtung Vorrats- und Entsorgungsbehältnis 22 bewegt wird. Die Kanüle 2 ist magnetisiert. Wenn der Führungsdraht 2 im Blutgefäß 1 positioniert ist, wird die magnetisierte Kanüle 2 mittels einer Magneteinrichtung 28 in Bewegung versetzt und durch die geöffnete Halteeinrichtung 17 und den Katheter 4 in Richtung des Pfeils B hindurch zum Vorrats- und Entsorgungsbehältnis 22 befördert. Die Magneteinrichtung 28 ist hier als ringförmiger Dauermagnet ausgeführt. Die Fig. 10 und 11 zeigen den Weg der Kanüle 2 zum Vorrats- und Entsorgungsbehältnis 22.

Wenn die Kanüle 2 entsorgt ist und auch der Führungsdraht 3 zurückgezogen ist und der Zugang des Katheters 4 zum Blutgefäß 1 gelegt ist, ist die Spritze 5 nur noch Ballast und wird nicht mehr benötigt. Mittels eines speziellen Werkzeuges 29 erfolgt die Abtrennung der Spritze 5 von der Vorrichtung. Das Werkzeug 29 weist zwei über ein Scharnier 30 verbundenen Backen 31 zum Umgreifen der Spritze 5 auf. An der Innenseite der Backen 31 sind nach innen weisende Zerkleinerungseinrichtungen 32 vorgesehen. Die Backen 31 sind Bestandteile einer Zange und sind über je einen Zangenhebel 33 gemäß den Pfeilen C aufeinander zu bewegbar, wobei die Zerstörung der Spritze 5 stattfindet, oder entgegen den Pfeilen C voneinander weg bewegbar.

Die Backen 31 sind Formteile sind, die auf den Tubus 13 mit der Halteeinrichtung 17 und den Kolben 11 mit der Verschlusseinrichtung 20 der Spritze 5 genau abgestimmt sind. Die Spritze 5 mit all ihren Bestandteilen besteht aus leicht zerstörbaren Materialien, hier aus Kunststoff.

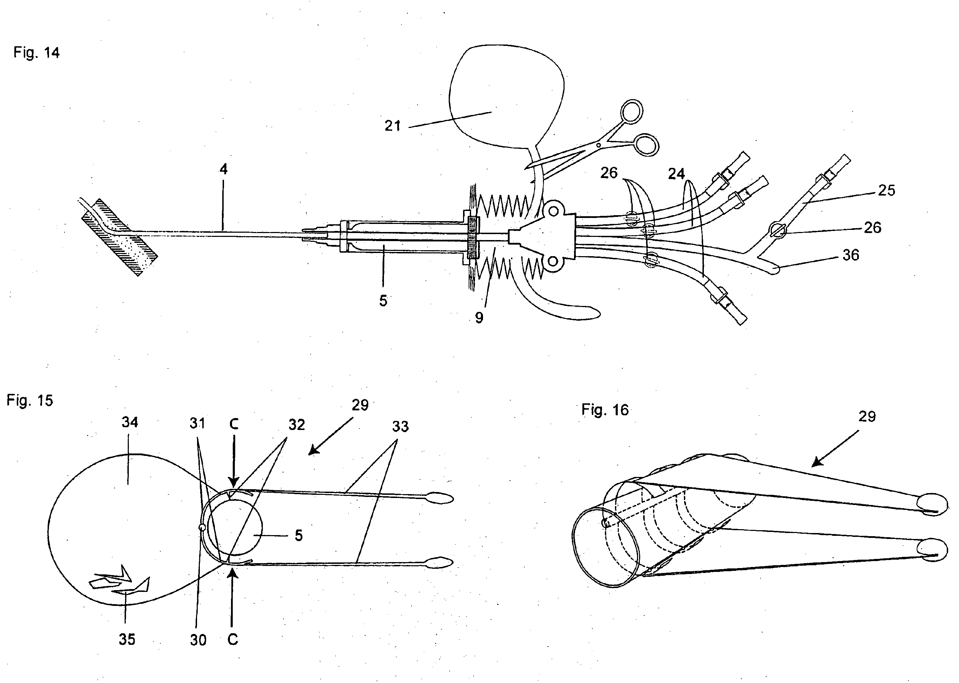

Aus Fig. 15 ist ersichtlich, dass das Werkzeug 29 eine Auffangeinrichtung 34 für die Bruchstücke 35 der zu zerstörenden Spritze 5 aufweist. Der Zerstörungsvorgang soll darin resultieren, dass der Katheter 4 von der Spritze 5 befreit ist. Auch die Umhüllung 9 mit der Druckausgleicheinrichtung 21 wird entfernt - hier gemäß Fig. 14 mittels nicht näher bezeichneter Schere.

Zum Anlegen des Zugangs zum Blutgefäß werden folgende Verfahrensschritte durchgeführt, die anhand der Figuren 3 bis 15 gut nachvollziehbar sind:
In Fig. 3 befindet sich die erfindungsgemäße Vorrichtung noch in Ruhestellung. Die Umhüllung 7 der Kanüle 2 ist noch intakt. Die Kanüle 2 sitzt fest und dicht in der Halteeinrichtung 17.

In Fig. 4 ist gezeigt, dass die Umhüllung 7 perforiert und bis zur Halteeinrichtung 17 zurückgeschoben wurde, das Blutgefäß 1 angestochen wurde und das Sterilpflaster 14 über die Einstichstelle gelegt wurde. Die im Tubus 13 der Spritze 5 vorhandene Kochsalzlösung wird in das Blutgefäß 1 injiziert, indem der Kolben 11 betätigt wird, wobei der Faltenbalg der Umhüllung 8 zusammengeschoben und die dortige sterile Luft aus der Umhüllung 8 in die Druckausgleicheinrichtung 21 verdrängt wird.

Fig. 5 zeigt, dass der Kolben 11 mit der Durchtrittsöffnung 6 direkt an die Durchtrittsöffnung 12 der Halteeinrichtung 17 für die Kanüle 2 anschließt. Es besteht eine Verbindung zwischen der Durchtrittsöffnung 6 des Kolbens 11 und der Kanüle 2, die dicht und fest in der Halteeinrichtung 17 sitzt, so dass keine Kontamination stattfinden kann. Die am Ende des Kolbens 11 befindliche Verschlusseinrichtung 20 ist während dieses Injektionsvorganges geschlossen und wird danach geöffnet, um den Führungsdraht 3 durch die Durchtrittsöffnung 6 und die Kanüle 2 bis in das Blutgefäß 1 hineintransportieren zu können. Der Vorschub wird über die Handhabungszone H in Richtung des Pfeils A realisiert.

In den Fig. 6 bis 8 geht es um die Aufweitung des Eingangs zum Blutgefäß 1 mittels Dilatator 16. Der Dilatator 16 weist ein hier nicht besonders dargestelltes Außengewinde auf, das mit dem Innengewinde des Gewindebauteils 19 an der Halteeinrichtung 17 zusammenwirkt. Das Gewinde weist nur einen Gewindegang auf und ist leicht von außen - über das Sterilpflaster 14 und die zurückgeschobene Umhüllung 7 zu betätigen. Der Dilatator wird 16 wird über die Kanüle 2 in Richtung A zur Einstichstelle und zum Blutgefäß 1 geschoben. Nach der Erweiterung wird der Dilatator 16 auf der Kanüle 2 in Richtung des Pfeils B zurückgeschoben und an der geschlossenen Halteeinrichtung 17 befestigt. Die Verschlusseinrichtung 20 kann während dieser Vorgänge geöffnet bleiben, zumal die Haltevorrichtung 17 geschlossen ist und die Kanüle 2 dicht und fest in dieser sitzt, so dass von außen keine Kontamination erfolgen kann.

Nachdem der Erweiterungsvorgang beendet ist und der Dilatator 16 wieder am Gewindebauteil 19 befestigt ist, wird der Katheter 4 in Richtung des Pfeils A aus der Umhüllung 9 durch die geöffnete Verschlusseinrichtung 20 und durch die Durchtrittsöffnung 6 der Spritze 5 bis in den Tubus 13, bis unmittelbar vor die geschlossene Halteeinrichtung 17, vorgeschoben. Es handelt sich hier um einen Katheter 4 mit vier Lumen. Das zentrale Lumen korrespondiert mit der Kanüle 2 und dem Vorrats- und Entsorgungsbehältnis 22, damit diese dann in einem nächsten Schritt durch den Katheter 4 in Richtung des Pfeils B abgeführt werden kann.

Das Zurückbewegen der Kanüle 2 in Richtung Pfeil B ist in den Fig. 10 und 11 gezeigt. Die Halteeinrichtung 17 wird geringfügig geöffnet und die magnetisierte Kanüle 2 wird mittels Magneteinrichtung 28 entlang des Führungsdrahtes 3 und durch den Katheter 4 hindurch zum Vorrats- und Entsorgungsbehältnis 22 bewegt. Das Vorratsund Entsorgungsbehältnis 22 stellt im vorliegenden Ausführungsbeispiel quasi eine Erweiterung des zentralen Lumens des Katheters 4 dar. Während der Kanülenentfernung ist die Halteinrichtung 17 zwangsläufig geöffnet, so dass Umgebungsluft eindringen kann. Das Sterilpflaster 14 kann natürlich keine absolute Sterilität gewährleisten. Deshalb wird über ein über den Anschluss 27 an die Umhüllung 9 angeschlossenes, hier nicht gezeigtes Pumpsystem sterile Luft in die Umhüllungen 9 und über die offene Verschlusseinrichtung 20 in die Umhüllung 8 bzw. in den Tubus 13 eingebracht. Es wird eine in Richtung des Pfeils A gerichtete Strömung, ein leichter Überdruck erzeugt, um das Eindringen kontaminierter Luft aus dem Bereich zwischen Tubus 13 bzw. Halteeinrichtung 17 und Blutgefäß 1 in den Katheter 4 zu vermeiden. Dabei findet der Druckausgleich innerhalb des Systems über die Druckausgleicheinrichtungen 21 statt.

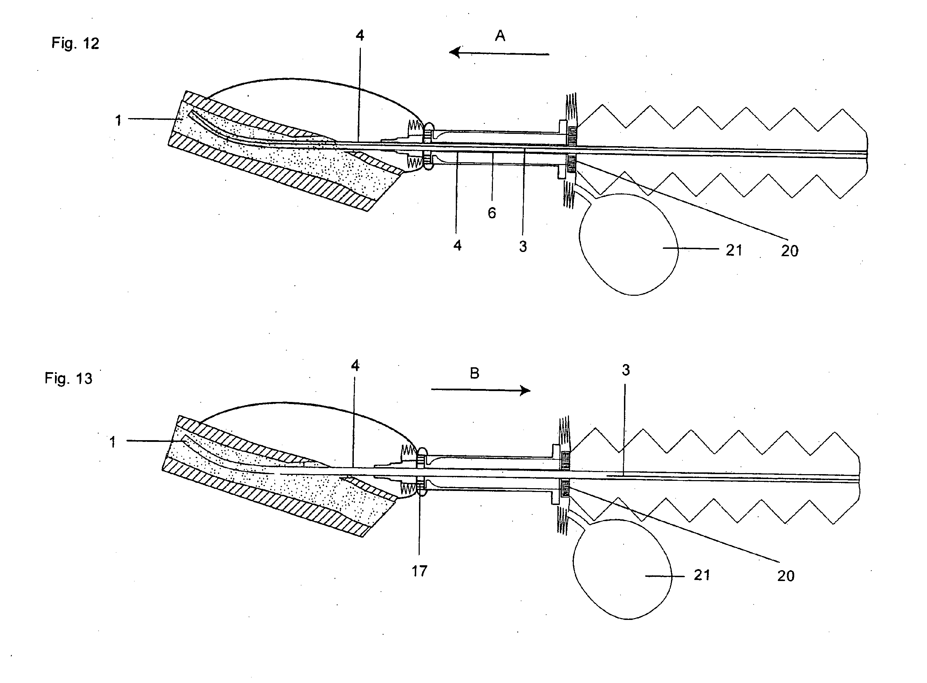

Nach der Entsorgung der Kanüle 2 im Vorrats- und Entsorgungsbehältnis 22 wird dann der Katheter 4 in Richtung des Pfeiles A entlang des Führungsdrahtes 3 durch die offene Verschlusseinrichtung 20, durch die Durchtrittsöffnung 6 der Spritze 5 und durch die offene Halteeinrichtung 17 hindurch in das Blutgefäß 1 bis zur gewünschten Position vorgeschoben, was in Fig. 12 dargestellt ist.

In Fig. 13 ist gezeigt, dass nach der Positionierung des Katheters 4 im Blutgefäß 1 der Führungsdraht 3 aus dem Blutgefäß 1 entfernt wird und in Richtung des Pfeils B in das Vorrats- und Entsorgungsbehältnis 22 verbracht wird.

Die Fig. 14 bis 16 zeigen die Nachbereitungsschritte, nachdem der Zugang gelegt ist. Zum einen wird das Vorrats- und Entsorgungsbehältnis 22 mit der entsorgten Kanüle 2 und dem entsorgten Führungsdraht 3 vom Katheter 4 steril abgeschweißt und so ein zusätzlicher Anschluss 36 erzeugt, durch den Kochsalzlösung oder Medikamente eingeführt werden können. Mittels Werkzeug 29 wird die Spritze 5 entfernt. Reste der Umhüllung 9 und dem Vorrats- und Entsorgungsbehältnis werden ebenfalls entfernt.

Hinsichtlich weiterer, in den Figuren nicht gezeigter Merkmale wird auf den allgemeinen Teil der Beschreibung verwiesen.

Abschließend sei darauf hingewiesen, dass die erfindungsgemäße Lehre nicht auf das voranstehend erörterte Ausführungsbeispiel eingeschränkt ist, sondern auf die beigefügten Ansprüche.

### Bezugszeichenliste

| | | | | |
|---|---|---|---|---|
| 1 | Blutgefäß | | 30 | Scharnier |
| 2 | Kanüle | | 31 | Backen |
| 3 | Führungsdraht | | 32 | Zerkleinerungseinrichtungen |
| 4 | Katheter | | 33 | Zangenhebel |
| 5 | Spritze | | 34 | Auffangeinrichtung |
| 6 | Durchtrittsöffnung | | 35 | Bruchstücke |
| 7 | Umhüllung | | 36 | Anschluss |
| 8 | Umhüllung | | | |
| 9 | Umfüllung | | | |
| 10 | Perforierungsbereich | | | |
| 11 | Kolben | | | |
| 12 | Durchtrittsöffnung | | | |
| 13 | Tubus | | | |
| 14 | Sterilpflaster | | | |
| 15 | Schutzhülse | | | |
| 16 | Dilatator | | | |
| 17 | Halteeinrichtung | | | |
| 18 | Umhüllung | | | |
| 19 | Gewindebauteil | | | |
| 20 | Verschlusseinrichtung | | | |
| 21 | Druckausgleicheinhchtung Verbindungsmittel | | | |
| 22 | Vorrats- und Entsorgungsbehättnis | | | |
| 23 | Verteiler | | | |
| 24 | Anschluss | | | |
| 25 | Anschluss | | | |
| 26 | Ventil | | | |
| 27 | Anschluss | | | |
| 28 | Magneteinrichtung | | | |
| 29 | Werkzeug | | | |
| | | | | |
| | A | Pfeil | | |
| | B | Pfeil | | |
| | C | Pfeil | | |
| | H | Handhabungszone | | |

## Patentansprüche

1. Vorrichtung zum Anlegen eines Zugangs zu einem Hohlorgan mit einer Kanüle (2), mit einem Führungsdraht (3) und mit einem Katheter (4),
**dadurch gekennzeichnet,**
**dass** die Kanüle (2) Bestandteil einer Spritze (5) mit einer Durchtrittsöffnung (6) für den Führungsdraht (3) und den Katheter (4) ist und dass die Spritze (5), der Katheter (4) und der Führungsdraht (3) innerhalb eines geschlossenen Systems in Form zumindest einer Umhüllung (7, 8, 9) angeordnet sind, wobei die Vorrichtung von außen, über die Umhüllung betätigbar ist, und dass die Umfüllung (7) in ihrem Perforationsbereich (10) durch die Kanüle (2) perforierbar ist.

2. Vorrichtung nach Anspruch 1, **dadurch gekennzeichnet, dass** sich die Durchtrittsöffnung (6) durch den Kolben (11) der Spritze (5) erstreckt und mit der kanüfenseitigen Durchtrittsöffnung (12) des Tubus (13) der Spritze (5) korrespondiert.

3. Vorrichtung nach Anspruch 1 oder 2, **dadurch gekennzeichnet, dass** mehrere Umhüllungen (8, 9) vorgesehen sind, die untereinander in Verbindung bringbar sind.

4. Vorrichtung nach einem der Ansprüche 1 bis 3, **dadurch gekennzeichnet, dass** die Umfüllung (7) im Perforierungsbereich (10) aus Latex besteht.

5. Vorrichtung nach einem der Ansprüche 1 bis 4, **dadurch gekennzeichnet, dass** an der Spritze (5) ein Sterilpflaster (14) vorgesehen ist, welches zur Abdeckung der Einstichstelle dient.

6. Vorrichtung nach Anspruch 4, **dadurch gekennzeichnet, dass** die Kanüle (2) und die dortige Umhüllung (7) während des Ruhezustandes innerhalb einer entfernbaren Schutzhülse (15) angeordnet sind.

7. Vorrichtung nach einem der Ansprüche 1 bis 6, **dadurch gekennzeichnet, dass** die Umhüllung (7) im Bereich der Kanüle (2) als Faltenbalg ausgebildet ist und nach der Perforation zurückschiebbar ist.

8. Vorrichtung nach einem der Ansprüche 1 bis 7, **dadurch gekennzeichnet, dass** die Spritze (5) einen Dilatator (16) umfasst.

9. Vorrichtung nach einem der Ansprüche 1 bis 8, **dadurch gekennzeichnet, dass** die Spritze (5) an ihrem Tubus (13) eine Hafteeinrichtung (17) für die Kanüle (2) umfasst, die vorzugsweise auf verschiedene Querschnitte, insbesondere auf den Querschnitt des Katheters (4), einstellbar ist.

10. Vorrichtung nach Anspruch 9, **dadurch gekennzeichnet, dass** die Halteeinrichtung (17) eine separate Umhüllung (18) aufweist.

11. Vorrichtung nach Ansprüche 9 oder 10 in Verbindung mit Anspruch 8, **dadurch gekennzeichnet, dass** der Dilatator (16) an der Halteeinrichtung (17) lösbar befestigt ist.

12. Vorrichtung nach einem der Ansprüche 3 bis 11, **dadurch gekennzeichnet, dass** die Umhüllung (8) der Spritze (5) im Bereich ihres Kolbens (11) zumindest teilweise als Faltenbalg ausgebildet ist.

13. Vorrichtung nach einem der Ansprüche 1 bis 12, **dadurch gekennzeichnet, dass** am Ende des Kolbens (11) der Spritze (5) eine Verschlusseinrichtung (20) vorgesehen ist, die die Durchtrittsöffnung (6) freigibt oder verschließt und die vorzugsweise auf verschieden Querschnitte des Führungsdrahtes (3) und des Katheters (4) einstellbar ist.

14. Vorrichtung nach einem der Ansprüche 3 bis 13, **dadurch gekennzeichnet, dass** eine Umhüllung (9) für den Führungsdraht (3) und den Katheter (4) vorgesehen ist, die vorzugsweise als Faltenbalg ausgeführt ist und sich an die Spritze (5) anschließt.

15. Vorrichtung nach Anspruch 3 bis 14, **dadurch gekennzeichnet, dass** die Umhüllung (8) der Spritze (5) im Bereich ihres Kolbens (11) und die Umhüllung (9) für den Führungsdraht (3) und den Katheter (4) jeweils eine Druckausgleicheinrichtung (21) umfassen.

16. Vorrichtung nach einem der Ansprüche 1 bis 15, **dadurch gekennzeichnet, dass** sich an die Umhüllung (9) für den Führungsdraht (3) und den Katheter (4) ein Vorratsund Entsorgungsbehältnis (22) anschließt, von dem aus Führungsdraht (3) und Katheter (4) zur Spritze (5) und schließlich zum Hohlorgan führbar sind.

17. Vorrichtung nach Anspruch 16, **dadurch gekennzeichnet, dass** der Katheter (4) bereits mit dem Führungsdraht (3) vorkonfektioniert ist.

18. Vorrichtung nach Anspruch 16 oder 17, **dadurch gekennzeichnet, dass** zwischen der Umhüllung (9) für den Führungsdraht (3) und den Katheter (4) und dem Vorrats- und Entsorgungsbehältnis (22) ein Verteiler (23) für diverse Anschlüsse (24, 25, 35) vorgese- hen ist.

19. Vorrichtung nach Anspruch 18, **dadurch gekennzeichnet, dass** den diversen, vorzugsweise steril konnektierbaren, Anschlüssen (24, 25) je ein steril ummanteltes Ventil (26) zugeordnet ist.

20. Vorrichtung nach einem der Ansprüche 1 bis 18, **dadurch gekennzeichnet, dass** die Umhüllung (9) einen steril konnektierbaren Anschluss (27) für ein Pumpsystem aufweist.

21. Vorrichtung nach einem der Ansprüche 1 bis 20, **dadurch gekennzeichnet, dass** die Kanüle (2) magnetisiert ist.

22. Vorrichtung nach Anspruch 21, **dadurch gekennzeichnet, dass** eine Magneteinrichtung (28) vorgesehen ist, die mit der magnetisierten Kanüle (2) zusammenwirkt und diese in Bewegung versetzt.

## Claims

1. A device for creating an access to a hollow organ, said device comprising a cannula (2), a guide wire (3), and a catheter (4),
**characterised in that**
the cannula (2) is a component of a syringe (5) having a passage opening (6) for the guide wire (3) and the catheter (4), and **in that** the syringe (5), the catheter (4), and the guide wire (3) are arranged inside a closed system in the form of at least one casing (7, 8, 9), wherein the device can be actuated from outside, via the casing, and **in that** the casing (7) can be perforated in its perforation zone (10) by the cannula (2).

2. The device according to Claim 1, **characterised in that** the passage opening (6) runs through the plunger (11) of the syringe (5) and communicates with the cannulaside passage opening (12) of the tube (13) of the syringe (5).

3. The device according to Claim 1 or 2, **characterised in that** multiple casings (8, 9) are provided that can be connected to one another.

4. The device according to one of Claims 1 to 3, **characterised in that** the casing (7) is made of latex in the perforation zone (10).

5. The device according to one of Claims 1 to 4, **characterised in that** a sterile patch (14) that is used to cover the puncture site is provided on the syringe (5).

6. The device according to Claim 4, **characterised in that** the cannula (2) and the casing (7) there are arranged inside a removable protective sleeve (15) when in the idle position.

7. The device according to one of Claims 1 to 6, **characterised in that** the casing (7) is designed as a bellows in the region of the cannula (2) and can be retracted after perforation.

8. The device according to one of Claims 1 to 7, **characterised in that** the syringe (5) comprises a dilator (16).

9. The device according to one of Claims 1 to 8, **characterised in that** the syringe (5) comprises, on its tube (13), a holding means (17) for the cannula (2) that can preferably be set to various cross sections, in particular to the cross section of the catheter (4).

10. The device according to Claim 9, **characterised in that** the holding means (17) comprises a separate casing (18).

11. The device according to Claims 9 or 10 in conjunction with Claim 8, **characterised in that** the dilator (16) is detachably attached to the holding means (17).

12. The device according to one of Claims 3 to 11, **characterised in that** the casing (8) of the syringe (5), in the region of the plunger (11) thereof, is at least partially formed as a bellows.

13. The device according to one of Claims 1 to 12, **characterised in that** a closing means (20) that opens or closes the passage opening (6) and can preferably be set to the various cross sections of the guide wire (3) and the catheter (4) is provided at the end of the plunger (11) of the syringe (5).

14. The device according to one of Claims 3 to 13, **characterised in that** a casing (9) for the guide wire (3) and the catheter (4) is provided that is preferably designed as a bellows and adjoins the syringe (5).

15. The device according to Claim 3 to 14, **characterised in that** the casing (8) of the syringe (5), in the region of the plunger (11) thereof, and the casing (9) for the guide wire (3) and the catheter (4), each comprise a pressure equalisation arrangement (21).

16. The device according to one of Claims 1 to 15, **characterised in that** the casing (9) for the guide wire (3) and the catheter (4) is adjoined by a storage and disposal container (22), from which the guide wire (3) and the catheter (4) can be conducted to the syringe (5) and eventually to the hollow organ.

17. The device according to Claim 16, **characterised in that** the catheter (4) is already preassembled with the guide wire (3).

18. The device according to Claim 16 or 17, **characterised in that** a manifold (23) for various ports (24, 25, 35) is provided between the casing (9) for the guide wire (3) and the catheter (4) and the storage and disposal container (22).

19. The device according to Claim 18, **characterised in that** a valve (26) having a sterile casing is associated with the various ports (24, 25), which preferably can be connected in a sterile manner.

20. The device according to one of Claims 1 to 18, **characterised in that** the casing (9) comprises a port (27) for a pumping system, said port being connectable in a sterile manner.

21. The device according to one of Claims 1 to 20, **characterised in that** the cannula (2) is magnetised.

22. The device according to Claim 21, **characterised in that** a magnetic means (28) is provided that interacts with the magnetised cannula (2) and sets the latter in motion.

## Revendications

1. Dispositif pour ménager un accès à un organe creux comprenant une canule (2), comprenant un fil de guidage (3) et un cathéter (4),
**caractérisé en ce que**
la canule (2) fait partie d'une aiguille (5) comprenant une ouverture de passage (6) pour le fil de guidage (3) et le cathéter (4) et que l'aiguille (5), le cathéter (4) et le fil de guidage (3) sont disposés à l'intérieur d'un système fermé sous forme d'au moins une enveloppe (7, 8, 9), sachant que le dispositif peut être actionné par l'extérieur, via l'enveloppe, et que l'enveloppe (7) peut être perforée par la canule (2) dans sa zone de perforation (10).

2. Dispositif selon la revendication 1, **caractérisé en ce que** l'ouverture de passage (6) s'étend à travers le piston (11) de l'aiguille (5) et correspondant avec l'ouverture de passage (12) côté canule du tube (13) de l'aiguille (5).

3. Dispositif selon la revendication 1 ou 2, **caractérisé en ce que** plusieurs enveloppes (8, 9) sont prévues qui peuvent être mises en liaison entre elles.

4. Dispositif selon l'une des revendications 1 à 3, **caractérisé en ce que** l'enveloppe (7) dans la zone de perforation (10) est en latex.

5. Dispositif selon l'une des revendications 1 à 4, **caractérisé en ce qu'**un pansement stérile (14) est prévu sur l'aiguille (5), lequel sert à recouvrir le point de piqûre.

6. Dispositif selon la revendication 4, **caractérisé en ce que** la canule (2) et l'enveloppe (7) qui s'y trouve sont disposées à l'intérieur d'un manchon de protection (15) amovible lorsqu'elles sont au repos.

7. Dispositif selon l'une des revendications 1 à 6, **caractérisé en ce que** l'enveloppe (7) est formée en tant que soufflet au niveau de la canule (2) et peut être rentrée après la perforation.

8. Dispositif selon l'une des revendications 1 à 7, **caractérisé en ce que** l'aiguille (5) comprend un dilatateur (16).

9. Dispositif selon l'une des revendications 1 à 8, **caractérisé en ce que** l'aiguille (5) comprend un dispositif de fixation (17) pour la canule (2) sur son tube (13), lequel est réglable de préférence à différentes sections transversales, en particulier à la section transversale du cathéter (4).

10. Dispositif selon la revendication 9, **caractérisé en ce que** le dispositif de fixation (17) présente une enveloppe (18) séparée.

11. Dispositif selon la revendication 9 ou 10 en liaison avec la revendication 8, **caractérisé en ce que** le dilatateur (16) est fixé de façon séparable sur le dispositif de fixation (17).

12. Dispositif selon l'une des revendications 3 à 11, **caractérisé en ce que** l'enveloppe (8) de l'aiguille (5) est formée au moins partiellement en tant que soufflet au niveau de son piston (11).

13. Dispositif selon l'une des revendications 1 à 12, **caractérisé en ce qu'**un dispositif de fermeture (20) est prévu sur l'extrémité du piston (11) de l'aiguille (5), qui libère ou ferme l'ouverture de passage (6) et qui est réglable de préférence à différentes sections transversales du fil de guidage (3) et du cathéter (4).

14. Dispositif selon l'une des revendications 3 à 13, **caractérisé en ce qu'**une enveloppe (9) pour le fil de guidage (3) et le cathéter (4) est prévue, laquelle est conçue de préférence en tant que soufflet et se raccorde sur l'aiguille (5).

15. Dispositif selon les revendications 3 à 14, **caractérisé en ce que** l'enveloppe (8) de l'aiguille (5), au niveau de son piston (11), et l'enveloppe (9) pour le fil de guidage (3) et le cathéter (4), comprennent respectivement un dispositif de compensation en pression (21).

16. Dispositif selon l'une des revendications 1 à 15, **caractérisé en ce qu'**un récipient de réserve et d'évacuation (22) se raccorde à l'enveloppe (9) pour le fil de guidage (3) et le cathéter (4), duquel le fil de guidage (3) et le cathéter (4) peuvent être guidés vers l'aiguille (5) et enfin vers l'organe creux.

17. Dispositif selon la revendication 16, **caractérisé en ce que** le cathéter (4) est déjà pré-confectionné avec le fil de guidage (3).

18. Dispositif selon la revendication 16 ou 17, **caractérisé en ce qu'**un distributeur (23) pour divers raccords (24, 25, 35) est prévu entre l'enveloppe (9) pour le fil de guidage (3) et le cathéter (4) et le récipient de réserve et d'évacuation (22).

19. Dispositif selon la revendication 18, **caractérisé en ce qu'**une valve (26) enveloppée de façon stérile est attribuée aux divers raccords (24, 25) pouvant de préférence être raccordés de façon stérile.

20. Dispositif selon l'une des revendications 1 à 18, **caractérisé en ce que** l'enveloppe (9) présente un raccord (27) pouvant être raccordé de façon stérile pour un système de pompe.

21. Dispositif selon l'une des revendications 1 à 20, **caractérisé en ce que** la canule (2) est aimantée.

22. Dispositif selon la revendication 21, **caractérisé en ce qu'**un dispositif d'aimant (28) est prévu qui coopère avec la canule (2) aimantée et la déplace.
